(19) Europäisches Patentamt
European Patent Office
Office européen des brevets

(11) Publication number: **0 354 766 B1**

(12) # EUROPEAN PATENT SPECIFICATION

(45) Date of publication of patent specification: **03.11.93**

(51) Int. Cl.[5]: **C07D 239/34**, A01N 43/54, C07D 239/38

(21) Application number: **89308060.6**

(22) Date of filing: **08.08.89**

(54) **5-substituted-2,4-diphenylpyrimidine derivatives, their production and herbicidal use.**

(30) Priority: **09.08.88 JP 199155/88**
**27.09.88 JP 243539/88**

(43) Date of publication of application:
**14.02.90 Bulletin 90/07**

(45) Publication of the grant of the patent:
**03.11.93 Bulletin 93/44**

(84) Designated Contracting States:
**CH DE ES FR GB IT LI**

(56) References cited:

**JOURNAL OF HETEROCYCLIC CHEMISTRY, vol. 23, no. 1, January/February 1986; M. TAKAHASHI et al., pp. 77-80&NUM;**

(73) Proprietor: **SUMITOMO CHEMICAL COMPANY, LIMITED**
**5-33 Kitahama 4-chome**
**Chuo-ku**
**Osaka-shi Osaka(JP)**

(72) Inventor: **Sato, Junichi**
**4-4-5, Kitajo-cho**
**Toyonaka-shi Osaka(JP)**
Inventor: **Sanemitsu, Yuzuru**
**2-2-924, Wakaba-cho**
**Ashiya-shi Hyogo(JP)**
Inventor: **Kawamura, Shinichi**
**2-7-508, Kokawa-cho**
**Chuo-ku**
**Osaka-shi Osaka(JP)**
Inventor: **Mito, Nobuaki**
**2-14-7, Mefu**
**Takarazuka-shi Hyogo(JP)**
Inventor: **Hamada, Tatsuhiro**
**2-14-7, Mefu**
**Takarazuka-shi Hyogo(JP)**
Inventor: **Yoshida, Ryo**
**3-10-63, Matsuzono-cho**
**Misawa-shi Aomori(JP)**

(74) Representative: **Diamond, Bryan Clive et al**
**Gee & Co.,**
**Chancery House,**
**Chancery Lane**
**London WC2A 1OU (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 354 766 B1

Rank Xerox (UK) Business Services
(3. 10/3.6/3.3. 1)

## Description

The present invention relates to 5-substituted-2,4-diphenylpyrimidine derivatives, their production and use. More particularly, it relates to novel 5-substituted-2,4-diphenylpyrimidine derivatives, a process for producing them, and their use as herbicides.

There have been used a number of herbicides. However, since they are insufficient for herbicidal effects, and also poor in the selectivity between crops and weeds, they are not always satisfactory.

The present inventors have made extensive and intensive studies to develop excellent herbicides, and consequently found that the following compound is good in herbicidal activity as well as in selectivity between crops and weeds. The present invention is based on this finding.

Namely, the compound of the present invention has the following structural formula;

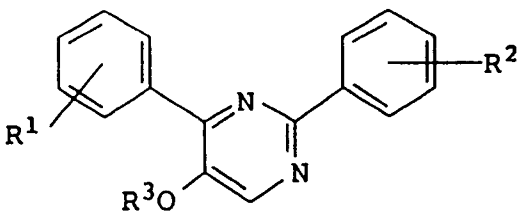

(I)

wherein $R^1$ represents a hydrogen atom, or a halogen atom, a ($C_1$ to $C_3$)-alkyl group, a halo-($C_1$ to $C_3$)-alkyl group, a ($C_1$ to $C_6$)-alkoxy group which may contain an unsaturated bond, a ($C_1$ to $C_2$)-alkylthio group, a halo-($C_1$ to $C_6$)-alkoxy group which may contain an unsaturated bond, a halo-($C_1$ to $C_2$)-alkylthio group, a phenoxy group, a ($C_1$ to $C_3$)-alkylcarboxy group, a halo-($C_1$ to $C_3$)-alkylcarboxy group, a ($C_1$ to $C_2$)-alkoxy-($C_1$ to $C_2$)-alkoxy group, a ($C_1$ to $C_3$)-alkylsulfonyloxy group, a halo-($C_1$ to $C_3$)-alkylsulfonyloxy group, a cyano group, a ($C_1$ to $C_3$)-alkoxycarbonyl group a hydroxycarbonyl group, an aminomethyl group or a hydroxymethyl group at the ortho or meta position; $R^2$ represents a hydrogen atom, a halogen atom, a ($C_1$ to $C_2$)-alkyl group, a halo-($C_1$ to $C_2$)-alkoxy group, a ($C_1$ to $C_2$)-alkoxy group, a nitro group, a ($C_1$ to $C_2$)-alkylthio group, a halo-($C_1$ to $C_2$)-alkylthio group or a halo-($C_1$ to $C_2$)-alkoxy group; and $R^3$ represents a ($C_1$ to $C_2$)-alkyl group, provided that both $R^1$ and $R^2$ are not a hydrogen atom at the same time.

J. Heterocyclic Chem., 23, 77 (1986) discloses 2,4-diphenyl-5-methanesulfonylpyrimidine, etc. similar to the compound of the present invention. However, in this paper, no mention is made of the biological activity of the compounds. Further, the paper does not disclose at all the chemical structure and biological activity of 5-substituted-2,4-diphenylpyrimidine derivatives of the present invention.

The compound of the present invention has an excellent herbicidal activity, and is good in the selectivity between crops and weeds. Namely, the compound of the present invention has a good herbicidal activity on a number of undesired weeds which will cause a problem to the foliar treatment as well as the soil treatment in upland fields. Examples of these weeds include broad-leaved weeds such as common chickweed (Stellaria media), radish (Raphanus sativus), wild mustard (Sinapis arvensis), velvetleaf (Abutilon theophrasti), prickly sida (Sida spinosa), field pansy (Viola arvensis), catchweed bedstraw (Galium aparine), ivyleaf morningglory (Ipomoea hederacea), tall morningglory (Pharbitis purpurea), black nightshade (Solanum nigrum), and persian speedwell (Veronica persica); and graminaceous weeds such as Japanese millet (Echinochloa frumentacea), barnyardgrass (Echinochloa crus-galli), green foxtail (Setaria viridis), large crabgrass (Digitaria sanguinalis), oats (Avena sativa), and wild oats (Avena fatua). In addition, the compound of the present invention does not exert undesired phytotoxicity to main crops such as wheat, barley, rice plant, soybean, cotton, and corn.

Further, the compound of the present invention has a herbicidal activity on a variety of weeds in paddy fields, for example, graminaceous weeds such as barnyardgrass (Echinochloa oryzoides), and does not exert undesired phytotoxicity on rice plant.

In the compound of the present invention, it is preferable that $R^1$ is a hydrogen atom, or a halogen atom, a ($C_1$ to $C_3$)-alkyl group, a halo-($C_1$ to $C_3$)-alkyl group, a ($C_1$ to $C_6$)-alkoxy group which may contain an unsaturated bond, a ($C_1$ to $C_2$)-alkylthio group, a halo-($C_1$ to $C_6$)-alkoxy group which may contain an unsaturated bond, a halo-($C_1$ to $C_2$)-alkylthio group, a phenoxy group, a ($C_1$ to $C_3$)-alkylcarboxy group, a halo-($C_1$ to $C_3$)-alkylcarboxy group, a ($C_1$ to $C_2$)-alkoxy-($C_1$ to $C_2$)-alkoxy group, a ($C_1$ to $C_3$)-alkylsulfonyloxy group, a halo-($C_1$ to $C_3$)-alkylsulfonyloxy group or a cyano group at the meta position; and $R^2$ is a hydrogen atom, or a halogen atom, a ($C_1$ to $C_2$)-alkyl group, a halo-($C_1$ to $C_2$)-alkyl group or a ($C_1$ to $C_2$)-alkoxy group at the meta or para position. More preferably, $R^1$ is a hydrogen atom, or a halogen atom, a trifluoromethyl group or a trifluoromethoxy group at the meta position; $R^2$ is a hydrogen atom, or a halogen

atom or a trifluoromethyl group at the para position; and $R^3$ is a methyl group. Most preferably, $R^1$ is a trifluoromethyl group or a trifluoromethoxy group at the meta position; $R^2$ is a fluorine atom, a chlorine atom or a trifluoromethyl group at the para position; and $R^3$ is a methyl group.

The particularly preferred compounds of the present invention include 2-(4-fluorophenyl)-4-(3-trifluoromethylphenyl)-5-methoxypyrimidine; 2-(4-trifluoromethylphenyl)-4-(3-trifluoromethoxyphenyl)-5-methoxypyrimidine; and 2-(4-fluorophenyl)-4-(3-trifluoromethoxyphenyl)-5-methoxypyrimidine.

The compound (I) of the present invention can be produced by the following procedures:

Procedure (A):

The compound (I) is prepared by reacting a compound of the formula:

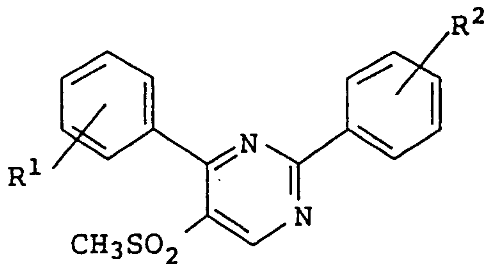

(II)

[wherein $R^1$ and $R^2$ are each as defined above] with a compound of the formula:

$R^3OM$ (III)

wherein M is a metal atom, and $R^3$ is as defined above].

The reaction is usually carried out in an inert solvent at a temperature of about 20 to 100 °C for a period of about 0.5 to 5 hours.

Normally, the compound (III) is used in an amount of about 1 to 10 equivalents to one equivalent of the compound (II).

The solvent includes aliphatic hydrocarbons (e.g., hexane, heptane, ligroin, petroleum ether), aromatic hydrocarbons (e.g., benzene, toluene, xylene), ethers (e.g., diethyl ether, diisopropyl ether, dioxane, tetrahydrofuran, diethylene glycol dimethyl ether), alcohols (e.g., methanol, ethanol, isopropanol, t-butanol), esters (e.g., ethyl acetate, butyl acetate), acid amides (e.g., N,N-dimethylformamide, acetamide), sulfur compounds (e.g., dimethyl sulfoxide), and mixtures thereof.

The compound (III) includes alkali metal alkoxides (e.g., sodium methoxide, sodium ethoxide).

After completion of the reaction, the reaction mixture is subjected to the usual after-treatment such as extraction with organic solvents, concentration, etc., to obtain the compound (I) of the present invention, and if necessary, purified by chromatography, distillation, recrystallization, etc.

Procedure (B):

A compound of the formula:

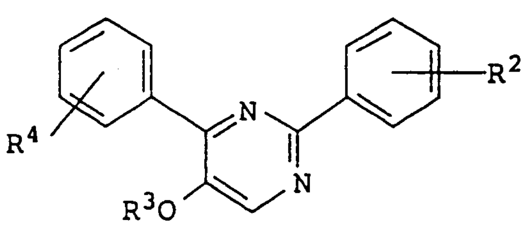

(I-1)

[wherein $R^4$ is a ($C_2$ to $C_6$)-alkoxy group or a halo-($C_2$ to $C_6$)-alkoxy group at the ortho or meta position; and $R^2$ and $R^3$ are each as defined above] is prepared by reacting a compound of the formula:

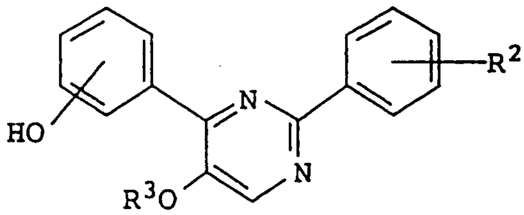

(IV)

[wherein $R^2$ and $R^3$ are each as defined above; and the hydroxyl group of the phenol moiety bonded to the pyrimidine ring is positioned at the ortho or meta position] with a ($C_2$ to $C_6$)-olefin which may be substituted with halogen.

The reaction is usually carried out in the presence of a base in an inert solvent at a temperature of 30 to 150 °C for a period of about 1 to 100 hours.

Normally, the olefin and the base are used respectively in amounts of about 5 to 30 equivalents and of about 1 to 10 equivalents to one equivalent of the compound (IV).

The solvent includes aliphatic hydrocarbons (e.g., hexane, heptane, ligroin, petroleum ether), aromatic hydrocarbons (e.g., benzene, toluene, xylene), halogenated hydrocarbons (e.g., chlorobenzene, dichlorobenzene), ethers (e.g., diethyl ether, diisopropyl ether, dioxane, tetrahydrofuran, diethylene glycol dimethyl ether), nitriles (e.g., acetonitrile, isobutyronitrile), acid amides (e.g., N,N-dimethylformamide, acetamide), water, and mixtures thereof.

The base includes inorganic bases (e.g., sodium hydroxide, potassium hydroxide, sodium carbonate, potassium carbonate, sodium hydride), etc.

After completion of the reaction, the reaction mixture is subjected to the usual after-treatment such as extraction with organic solvents, concentration, etc., and if necessary, purified by chromatography, distillation, recrystallization, etc.

Procedure (C):

A compound of the formula:

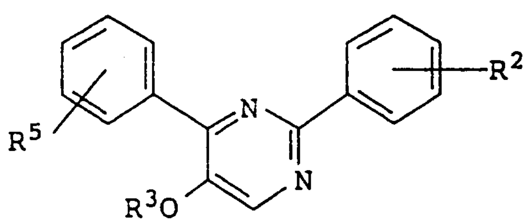

(I-2)

[wherein $R^5$ is a ($C_1$ to $C_3$)-alkylsulfonyloxy group or a halo-($C_1$ to $C_3$)-alkylsulfonyloxy group at the ortho or meta position; and $R^2$ and $R^3$ are each as defined above] is prepared by reacting a compound of the formula:

$R^6$-Cl (V)

[wherein $R^6$ is a ($C_1$ to $C_3$)-alkylsulfonyl group which may be substituted with halogen] with the compound (IV).

The reaction is usually carried out with or without an inert solvent and in the presence of a base at a temperature of about 0 to 50 °C for a period of about 0.5 to 5 hours.

Normally, the compound (V) and the base are used respectively in amounts of about 1 to 2 equivalents and of about 1 to 5 equivalents to one equivalent of the compound (IV).

The solvent includes aliphatic hydrocarbons (e.g., hexane, heptane, ligroin, petroleum ether), aromatic hydrocarbons (e.g., benzene, toluene, xylene), halogenated hydrocarbons (e.g., chloroform, carbon tetrachloride, dichloroethane, chlorobenzene, dichlorobenzene), ethers (e.g., diethyl ether, diisopropyl ether, dioxane, tetrahydrofuran, diethylene glycol dimethyl ether), ketones (e.g., acetone, methyl ethyl ketone, methyl isobutyl ketone, isophorone, cyclohexanone), esters (e.g., ethyl formate, ethyl acetate, butyl acetate, diethyl carbonate), pyridine, acid amides (e.g., N,N-dimethylformamide, acetamide), water, and mixtures

thereof.

The base includes organic bases (e.g., pyridine).

After completion of the reaction, the reaction mixture is subjected to the usual after-treatment such as extraction with organic solvents, concentration, etc., and if necessary, purified by chromatography, distillation, recrystallization, etc.

Procedure (D):

A compound of the formula:

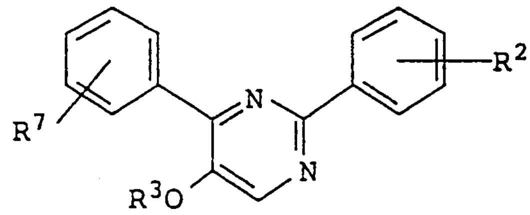

(I-3)

[wherein $R^7$ is a ($C_1$ to $C_6$)-alkoxy group which may contain an unsaturated bond, at the ortho or meta position; and $R^2$ and $R^3$ are each as defined above] is prepared by reacting a compound of the formula:

$R^8$-X (VI)

[wherein $R^8$ is a ($C_1$ to $C_6$)-alkyl group, a ($C_2$ to $C_6$)-alkenyl group or a ($C_2$ to $C_6$)-alkynyl group; and X is a halogen atom] with the compound (IV).

The reaction is usually carried out in the presence of a base in an inert solvent at a temperature of 20 to 150 °C for a period of about 0.5 to 50 hours.

Normally, the compound (VI) and the base are used respectively in amounts of about 1 to 20 equivalents and of about 1 to 20 equivalents to one equivalent of the compound (IV).

The solvent includes aliphatic hydrocarbons (e.g., hexane, heptane, ligroin, petroleum ether), aromatic hydrocarbons (e.g., benzene, toluene, xylene), ethers (e.g., diethyl ether, diisopropyl ether, dioxane, tetrahydrofuran, diethylene glycol dimethyl ether), ketones (e.g., acetone, methyl ethyl ketone, methyl isobutyl ketone, isophorone, cyclohexanone), esters (e.g., ethyl formate, ethyl acetate, butyl acetate, diethyl carbonate), nitriles (e.g., acetonitrile, isobutyronitrile), acid amides (e.g., N,N-dimethylformamide, acetamide), and water.

The base includes inorganic bases (e.g., sodium hydroxide, potassium hydroxide, sodium carbonate, potassium carbonate, sodium hydride).

After completion of the reaction, the reaction mixture is subjected to the usual after-treatment such as extraction with organic solvents, concentration, etc., and if necessary, purified by chromatography, distillation, recrystallization, etc.

Procedure (E):

A compound of the formula:

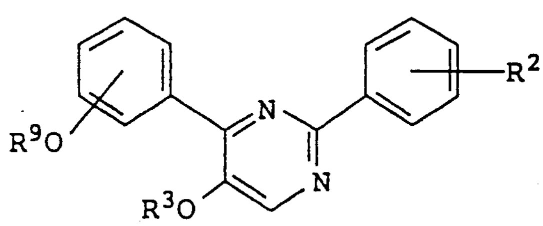

(I-4)

[wherein $R^9$ is a ($C_1$ to $C_3$)-acyl group; and $R^2$ and $R^3$ are each as defined above] is prepared by reacting a compound of the formula:

$R^9$-O-$R^9$ (VII)

[wherein $R^9$ is as defined above] with the compound (IV).

The reaction is usually carried out with or without an inert solvent and in the presence of an acid catalyst at a temperature of about 0 to 100 °C for a period of about 0.5 to 30 hours.

Normally, the compound (VII) is used in an amount of about 1 to 3 equivalents to one equivalent of the compound (IV).

The solvent includes aliphatic hydrocarbons (e.g., hexane, heptane, ligroin, petroleum ether), aromatic hydrocarbons (e.g., benzene, toluene, xylene), halogenated hydrocarbons (e.g., chloroform, carbon tetrachloride, dichloroethane, chlorobenzene, dichlorobenzene), ethers (e.g., diethyl ether, diisopropyl ether, dioxane, tetrahydrofuran, diethylene glycol dimethyl ether), ketones (e.g., acetone, methyl ethyl ketone, methyl isobutyl ketone, isophorone, cyclohexanone), acid amides (e.g., N,N-dimethylformamide, acetamide), water, and mixtures thereof.

The acid catalyst includes organic acids (e.g., acetic acid), inorganic acids (e.g., hydrochloric acid, sulfuric acid), etc.

After completion of the reaction, the reaction mixture is subjected to the usual after-treatment such as extraction with organic solvents, concentration, etc., and if necessary, purified by chromatography, distillation, recrystallization, etc.

Table 1 illustrates examples of the compound (I) of the present invention which can be produced by the above procedures.

Table 1

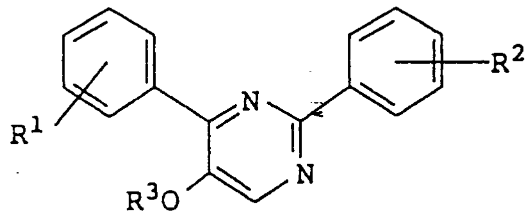

| $R^1$ | $R^2$ | $R^3$ |
|---|---|---|
| H | p-Cl | $CH_3$ |
| H | p-Br | $CH_3$ |
| H | p-$CF_3$ | $CH_3$ |
| H | p-$NO_2$ | $CH_3$ |
| m-F | H | $CH_3$ |
| m-F | p-$CH_3$ | $CH_3$ |
| m-F | p-F | $CH_3$ |
| m-F | p-$CF_3$ | $CH_3$ |
| m-F | p-Cl | $CH_3$ |
| m-F | p-Br | $CH_3$ |
| m-F | p-F | $C_2H_5$ |
| m-Cl | p-F | $CH_3$ |
| m-Cl | H | $CH_3$ |
| m-Cl | p-Cl | $CH_3$ |
| m-Cl | p-$CF_3$ | $CH_3$ |
| m-Br | H | $CH_3$ |

Table 1 (cont'd)

| $R^1$ | $R^2$ | $R^3$ |
|---|---|---|
| m-Br | p-Cl | $CH_3$ |
| m-Br | p-Br | $CH_3$ |
| m-Br | p-$CF_3$ | $CH_3$ |
| m-Br | p-$NO_2$ | $CH_3$ |
| m-Br | p-F | $C_2H_5$ |
| o-Br | p-$CF_3$ | $CH_3$ |
| m-I | p-$CF_3$ | $CH_3$ |
| m-I | p-F | $CH_3$ |
| m-I | p-F | $C_2H_5$ |
| m-$CH_3$ | p-F | $CH_3$ |
| m-$CH_3$ | p-$CF_3$ | $CH_3$ |
| o-$CH_3$ | p-$CF_3$ | $CH_3$ |
| m-$CF_3$ | H | $CH_3$ |
| m-$CF_3$ | p-$CH_3$ | $CH_3$ |
| m-$CF_3$ | p-F | $CH_3$ |
| m-$CF_3$ | p-Cl | $CH_3$ |
| m-$CF_3$ | p-Br | $CH_3$ |
| m-$CF_3$ | p-$CF_3$ | $CH_3$ |
| m-$CF_3$ | p-$CH_3O$ | $CH_3$ |
| m-$CF_3$ | m-$CH_3O$ | $CH_3$ |
| m-$CF_3$ | m-F | $CH_3$ |

Table 1 (cont'd)

| $R^1$ | $R^2$ | $R^3$ |
|---|---|---|
| m-$CF_3$ | o-F | $CH_3$ |
| m-$CF_3$ | p-F | $C_2H_5$ |
| m-$CF_3$ | p-$CF_3$ | $C_2H_5$ |
| m-$CF_3$ | p-Cl | $C_2H_5$ |
| m-$CF_3$ | p-$CH_3$ | $C_2H_5$ |
| m-$CF_3$ | H | $C_2H_5$ |
| o-$CF_3$ | p-$CF_3$ | $CH_3$ |
| o-$CF_3$ | o-F | $CH_3$ |
| m-$CH_3O$ | p-F | $CH_3$ |
| m-$CH_3O$ | p-$CF_3$ | $CH_3$ |
| m-$C_2H_5O$ | p-F | $CH_3$ |
| m-$C_2H_5O$ | p-$CF_3$ | $CH_3$ |
| m-(i)$C_3H_7O$ | p-F | $CH_3$ |
| m-(sec)$C_4H_9O$ | p-F | $CH_3$ |
| m-(sec)$C_4H_9O$ | p-$CF_3$ | $CH_3$ |
| m-$CH_2$=$CHCH_2O$ | p-F | $CH_3$ |
| m-$CH_2$=$CHCH_2O$ | p-$CF_3$ | $CH_3$ |
| m-$CH{\equiv}CCH_2O$ | p-F | $CH_3$ |
| m-$CH{\equiv}CCH_2O$ | p-$CF_3$ | $CH_3$ |
| m-$CH_3S$ | p-F | $CH_3$ |
| m-$CH_3S$ | p-$CF_3$ | $CH_3$ |
| m-$CF_3O$ | p-$CF_3$ | $CH_3$ |

Table 1 (cont'd)

| $R^1$ | $R^2$ | $R^3$ |
|---|---|---|
| m-$CF_3O$ | p-F | $CH_3$ |
| m-$CF_3O$ | p-Cl | $CH_3$ |
| m-$CF_3O$ | p-F | $C_2H_5$ |
| m-$CCl_3O$ | p-F | $CH_3$ |
| m-$CCl_3O$ | p-$CF_3$ | $CH_3$ |
| m-$CF_2HO$ | p-F | $CH_3$ |
| m-$CF_2HO$ | p-$CF_3$ | $CH_3$ |
| m-$CF_2ClO$ | p-F | $CH_3$ |
| m-$CF_2ClO$ | p-$CF_3$ | $CH_3$ |
| m-$CF_3CF_2O$ | p-F | $CH_3$ |
| m-$CF_3CF_2O$ | p-$CF_3$ | $CH_3$ |
| m-$CF_2HCF_2O$ | p-F | $CH_3$ |
| m-$CF_2HCF_2O$ | p-$CF_3$ | $CH_3$ |
| m-$CCl_2HCF_2O$ | p-F | $CH_3$ |
| m-$CCl_2HCF_2O$ | p-$CF_3$ | $CH_3$ |
| m-$CF_3S$ | p-F | $CH_3$ |
| m-$CF_3S$ | p-$CF_3$ | $CH_3$ |
| m-$C_6H_5O$ | p-F | $CH_3$ |
| m-$C_6H_5O$ | p-$CF_3$ | $CH_3$ |
| m-$CH_3CO_2$ | p-F | $CH_3$ |
| m-$CH_3CO_2$ | p-$CF_3$ | $CH_3$ |
| m-$CClFHCF_2O$ | p-F | $CH_3$ |

Table 1 (cont'd)

| $R^1$ | $R^2$ | $R^3$ |
|---|---|---|
| m-$CClFHCF_2O$ | p-$CF_3$ | $CH_3$ |
| m-CClFHCClFO | p-F | $CH_3$ |
| m-CClFHCClFO | p-$CF_3$ | $CH_3$ |
| m-$CF_3CO_2$ | p-F | $CH_3$ |
| m-$CH_3OCH_2O$ | p-F | $CH_3$ |
| m-$CH_3OCH_2O$ | p-$CF_3$ | $CH_3$ |
| m-$CH_3SO_3$ | p-F | $CH_3$ |
| m-$CH_3SO_3$ | p-$CF_3$ | $CH_3$ |
| m-$CH_3CH_2SO_3$ | p-F | $CH_3$ |
| m-$CH_3CH_2SO_3$ | p-$CF_3$ | $CH_3$ |
| m-$CF_3SO_3$ | p-F | $CH_3$ |
| m-$CF_3SO_3$ | p-$CF_3$ | $CH_3$ |
| m-$CF_3CF_2SO_3$ | p-F | $CH_3$ |
| m-$CF_3CF_2SO_3$ | p-$CF_3$ | $CH_3$ |
| m-$CF_3O$ | p-$CF_3O$ | $CH_3$ |
| m-$CF_3$ | p-$CF_3O$ | $CH_3$ |
| m-$CH_2NH_2$ | p-$CF_3$ | $CH_3$ |
| m-$CH_2NH_2$ | p-F | $CH_3$ |
| m-$CH_2OH$ | p-$CF_3$ | $CH_3$ |
| m-$CH_2OH$ | p-F | $CH_3$ |
| m-$CF_3O$ | p-$SCH_3$ | $CH_3$ |
| m-$CF_3$ | p-$SCH_3$ | $CH_3$ |

Table 1 (cont'd)

| $R^1$ | $R^2$ | $R^3$ |
|---|---|---|
| m-CN | p-$CF_3$ | $CH_3$ |
| m-CN | p-F | $CH_3$ |
| m-COOH | p-$CF_3$ | $CH_3$ |
| m-COOH | p-F | $CH_3$ |
| m-$COOCH_3$ | p-$CF_3$ | $CH_3$ |
| m-$COOCH_3$ | p-F | $CH_3$ |
| m-CClF=CFO | p-F | $CH_3$ |
| m-$CF_3O$ | p-$SCF_3$ | $CH_3$ |
| m-$CF_3$ | p-$SCF_3$ | $CH_3$ |

The present invention will be illustrated in more detail by the following examples, but the invention is not limited thereto.

First, Production Examples of the present invention are shown below.

Production Example 1

2 g of 2-(4-fluorophenyl)-4-(3-trifluoromethylphenyl)-5-methanesulfonylypyrimidine and 0.3 g of sodium methoxide were added to 20 ml of ethylene glycol dimethyl ether, and the resulting mixture was heated under reflux for 5 hours. The solvent was distilled away under reduced pressure. The residue was washed with water, and then dried to obtain 1.5 g of 2-(4-fluorophenyl)-4-(3-trifluoromethylphenyl)-5-methoxypyrimidine (Compound 18).

Production Example 2

1.5 g of 2-(4-fluorophenyl)-4-(3-hydroxyphenyl)-5-methoxypyrimidine and 0.25 g of sodium hydride were added to 15 ml of N,N-dimethylformamide, and the resulting mixture was heated to 70 ° C. After adding 6.6 g of 1,1-dichloro-2,2-difluoroethylene thereto, the mixture was heated under reflux for 3 days. After completion of the reaction, ice-cold water was added to the mixture. Subsequently the mixture was extracted with ethyl acetate. The solvent was evaporated off, and the residue was purified by column chromatography on silica gel, thereby obtaining 1.0 g of 2-(4-fluorophenyl)-4-[3-(1′,1′-difluoro-2′,2′-dichloroethoxy)phenyl]-5-methoxypyrimidine (Compound 53).

Production Example 3

1.2 g of 2-(4-fluorophenyl)-4-(3-hydroxyphenyl)-5-methoxypyrimidine and 0.57 g of methanesulfonyl chloride were added to 10 ml of pyridine, and the resulting mixture was stirred for 3 hours at room temperature. After completion of the reaction, ice-cold water was added to the mixture. Subsequently the mixture was extracted with ethyl acetate. The solvent was evaporated off, and the residue was purified by column chromatography on silica gel, thereby obtaining 1.0 g of 2-(4-fluorophenyl)-4-(3-methanesulfonyloxyphenyl)-5-methoxypyrimidine (Compound 54).

Production Example 4

1.2 g of 2-(4-fluorophenyl)-4-(3-hydroxyphenyl)-5-methoxypyrimidine, 5.5 g of potassium carbonate, and 5.5 g of 2-bromobutane were added to 200 ml of acetone, and the resulting mixture was heated under reflux for 2 days. The solvent was distilled away under reduced pressure. The residue was washed with water, and then purified by column chromatography on silica gel, thereby obtaining 1.0 g of 2-(4-fluorophenyl)-4-[3-*sec*-butoxyphenyl)-5-methoxypyrimidine (Compound 50).

Production Example 5

One drop of 96% sulfuric acid was added to a mixture of 1.2 g of 2-(4-fluorophenyl)-4-(3-hydroxyphenyl)-5-methoxypyrimidine and 0.5 g of acetic anhydride, and the resulting mixture was stirred for 20 hours at room temperature. After completion of the reaction, ice-cold water was added to the mixture. Subsequently the mixture was extracted with ethyl acetate. The solvent was evaporated off, and the residue was purified by column chromatography on silica gel, thereby obtaining 1.0 g of 2-(4-fluorophenyl)-4-(3-acetoxyphenyl)-5-methoxypyrimidine (Compound 55).

Table 2 illustrates part of the compounds of the present invention produced according to the above Production Examples.

Table 2

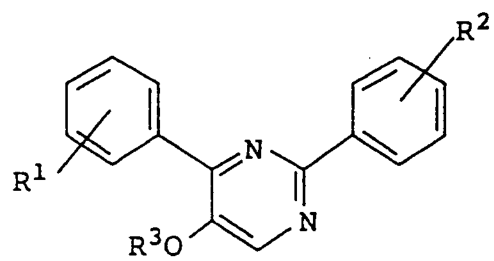

| Compound No. | $R^1$ | $R^2$ | $R^3$ | Melting Point (°C) |
|---|---|---|---|---|
| 1 | m-Cl | p-F | $CH_3$ | 126.0 |
| 2 | H | p-Cl | $CH_3$ | 105.5 |
| 3 | H | p-Br | $CH_3$ | 155.4 |
| 4 | H | p-$CF_3$ | $CH_3$ | 84.4 |
| 5 | H | p-$NO_2$ | $CH_3$ | 163.5 |
| 6 | m-Cl | H | $CH_3$ | 97.0 |
| 7 | m-Cl | p-Cl | $CH_3$ | 143.0 |
| 8 | m-Cl | p-$CF_3$ | $CH_3$ | 97.0 |
| 9 | m-Br | H | $CH_3$ | 103.2 |
| 10 | m-Br | p-Cl | $CH_3$ | 153.3 |

Table 2 (cont'd)

| Compound No. | $R^1$ | $R^2$ | $R^3$ | Melting Point (°C) |
|---|---|---|---|---|
| 11 | m-Br | p-Br | $CH_3$ | 158.9 |
| 12 | m-Br | p-$CF_3$ | $CH_3$ | 124.5 |
| 13 | m-Br | p-$NO_2$ | $CH_3$ | 152.1(dec) |
| 14 | o-Br | p-$CF_3$ | $CH_3$ | 119.0 |
| 15 | o-$CH_3$ | p-$CF_3$ | $CH_3$ | 103.4 |
| 16 | m-$CF_3$ | H | $CH_3$ | 92.6 |
| 17 | m-$CF_3$ | p-$CH_3$ | $CH_3$ | 95.8 |
| 18 | m-$CF_3$ | p-F | $CH_3$ | 98.9 |
| 19 | m-$CF_3$ | p-Cl | $CH_3$ | 127.4 |
| 20 | m-$CF_3$ | p-Br | $CH_3$ | 130.5 |
| 21 | m-$CF_3$ | p-$CF_3$ | $CH_3$ | 108.3 |
| 22 | m-$CF_3$ | p-$CH_3O$ | $CH_3$ | 104.3 |
| 23 | m-I | p-$CF_3$ | $CH_3$ | 131.4 |
| 24 | m-F | H | $CH_3$ | 83.5 |
| 25 | m-F | p-$CH_3$ | $CH_3$ | 72.5 |
| 26 | m-F | p-F | $CH_3$ | 98.3 |
| 27 | m-F | p-$CF_3$ | $CH_3$ | 101.3 |
| 28 | m-F | p-Cl | $CH_3$ | 104.5 |
| 29 | m-F | p-Br | $CH_3$ | 131.2 |
| 30 | o-$CF_3$ | p-$CF_3$ | $CH_3$ | 142.5 |
| 31 | m-$CF_3O$ | p-$CF_3$ | $CH_3$ | 94.6 |

Table 2 (cont'd)

| Compound No. | $R^1$ | $R^2$ | $R^3$ | Melting Point (°C) |
|---|---|---|---|---|
| 32 | m-$CF_3O$ | p-F | $CH_3$ | 73.9 |
| 33 | m-I | p-F | $CH_3$ | 111.5 |
| 34 | m-$CF_3$ | p-F | $C_2H_5$ | 90.6 |
| 35 | m-$CF_3$ | p-$CF_3$ | $C_2H_5$ | 101.4 |
| 36 | m-$CF_3$ | p-Cl | $C_2H_5$ | 103.0 |
| 37 | m-$CF_3$ | p-$CH_3$ | $C_2H_5$ | 98.4 |
| 38 | m-$CF_3$ | H | $C_2H_5$ | 104.1 |
| 39 | m-Br | p-F | $C_2H_5$ | 110.0 |
| 40 | m-F | p-F | $C_2H_5$ | 111.9 |
| 41 | m-I | p-F | $C_2H_5$ | 122.5 |
| 42 | m-$CF_3O$ | p-F | $C_2H_5$ | 66.6 |
| 43 | m-$CH_3$ | p-F | $CH_3$ | 106.7 |
| 44 | m-$C_2H_5O$ | p-F | $CH_3$ | 86.1 |
| 45 | m-$CH_3OCH_2O$ | p-F | $CH_3$ | 93.4 |
| 46 | m-$CH_3O$ | p-F | $CH_3$ | 90.2 |
| 47 | m-(i)$C_3H_7O$ | p-F | $CH_3$ | 78.2 |
| 48 | m-$CF_3O$ | p-Cl | $CH_3$ | 95.3 |
| 49 | m-$CF_2HCF_2O$ | p-F | $CH_3$ | 79.2 |
| 50 | m-(sec)$C_4H_9O$ | p-F | $CH_3$ | 86.0 |
| 51 | m-$C_6H_5O$ | p-F | $CH_3$ | 150.4 |
| 52 | m-$CF_3$ | o-F | $CH_3$ | 67.8 |

Table 2 (cont'd)

| Compound No. | $R^1$ | $R^2$ | $R^3$ | Melting Point (°C) |
|---|---|---|---|---|
| 53 | m-$CCl_2HCF_2O$ | p-F | $CH_3$ | 75.5 |
| 54 | m-$CH_3SO_3$ | p-F | $CH_3$ | 121.4 |
| 55 | m-$CH_3CO_2$ | p-F | $CH_3$ | 113.6 |
| 56 | m-$CF_3CO_2$ | p-F | $CH_3$ | 147.4 |
| 57 | m-$CF_3$ | m-$CH_3O$ | $CH_3$ | 83.1 |
| 58 | m-$CF_3$ | m-F | $CH_3$ | 60.0 |
| 59 | m-$CF_3SO_3$ | p-F | $CH_3$ | 127.5 |
| 60 | m-$CH_3O$ | p-$CF_3$ | $CH_3$ | 98.2 |
| 61 | m-$C_2H_5O$ | p-$CF_3$ | $CH_3$ | 86.4 |
| 62 | m-$CH_3OCH_2O$ | p-$CF_3$ | $CH_3$ | 79.3 |
| 63 | m-$C_6H_5O$ | p-$CF_3$ | $CH_3$ | 132.2 |
| 64 | m-$CF_3$ | m-$CF_3$ | $CH_3$ | 98.2 |
| 65 | m-$CF_3O$ | m-F | $CH_3$ | 51.6 |
| 66 | m-$CF_3O$ | m-$CF_3$ | $CH_3$ | 55.7 |
| 67 | m-$CH_2$=$CHCH_2O$ | p-F | $CH_3$ | 1.6180 ($n_D^{25}$) |
| 68 | m-$CF_3$ | m-Cl | $CH_3$ | 78.8 |
| 69 | m-$CF_2HO$ | p-$CF_3$ | $CH_3$ | 93.4 |
| 70 | m-$CF_3O$ | m-Cl | $CH_3$ | 77.8 |
| 71 | m-$CF_3$ | p-$SCH_3$ | $CH_3$ | 121.1 |
| 72 | m-CN | p-$CF_3$ | $CH_3$ | 131.7 |
| 73 | m-$CF_2HO$ | p-F | $CH_3$ | 88.5 |

The compound (II), which is a starting material for the compound of the present invention, is prepared by reacting a compound of the formula:

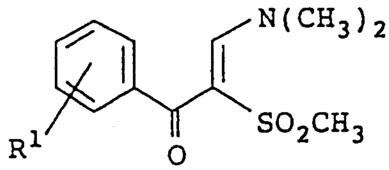

(VIII)

[wherein $R^1$ is as defined above] with a compound of the formula:

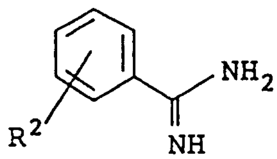

(IX)

[wherein $R^2$ is as defined above].

The reaction is usually carried out in a solvent at a temperature of about 20 to 100 ° C for a period of about 0.5 to 5 hours.

Normally, the compound (IX) is used in an amount of about 1.1 to 1.2 equivalents to one equivalent of the compound (VIII).

The solvent includes aliphatic hydrocarbons (e.g., hexane, heptane, ligroin, petroleum ether), aromatic hydrocarbons (e.g., benzene, toluene, xylene), halogenated hydrocarbons (e.g., chlorobenzene, dichlorobenzene), ethers (e.g., diethyl ether, diisopropyl ether, dioxane, tetrahydrofuran, diethylene glycol dimethyl ether), alcohols (e.g., methanol, ethanol, isopropanol), acid amides (e.g., N,N-dimethylformamide, acetamide), sulfur compounds (e.g., dimethyl sulfoxide, sulfolane), and mixtures thereof.

In the above reaction, the compound (IX) is normally used in the form of adducts of acids (e.g., a salt of hydrochloric acid). In this case, inorganic bases (e.g., potassium carbonate) or alkali metal alkoxides (e.g., sodium methoxide, sodium ethoxide) are used.

After completion of the reaction, the reaction mixture is subjected to the usual after-treatment such as extraction with organic solvents, concentration, etc., and if necessary, purified by chromatography, recrystallization, etc.

Production Example 6

6 g of 1-(3-trifluoromethylbenzoyl)-1-methanesulfonyl-2-(N,N-dimethylamino)ethene and 4 g of 4-fluorobenzamidine hydrochloride were dissolved in 30 ml of methanol at room temperature. After adding 1.2 g of sodium methoxide thereto, the mixture was heated under reflux for 1 hour. The solvent was distilled away under reduced pressure. The residue was washed with water, and then dried to obtain 6 g of 2-(4-fluorophenyl)-d-(3-trifluoromethylphenyl)-5-methanesulfonylpyrimidine (Compound 112).

Table 3 illustrates examples of the compound (II) produced according to the above Production Example 6.

Table 3

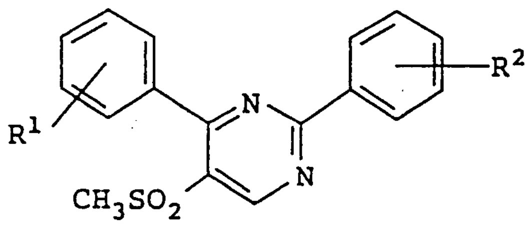

| Compound No. | $R^1$ | $R^2$ | $^1H$-NMR (δ value)* |
|---|---|---|---|
| 101 | H | p-$CF_3$ | 9.55 (s, 1H), 8.72-8.6(d, 2H), 8.05-7.5 (m, 7H),3.15 (s, 3H) |
| 102 | H | p-$NO_2$ | 9.45 (s,1H), 8.8-8.3 (q, 4H), 7.7 (brs, 5H), 3.2 (s, 3H) |
| 103 | m-Cl | H | 9.45 (s, 1H), 8.65-8.45 (m, 2H), 7.8-7.45 (m, 7H), 2.8 (s, 3H) |

Table 3 (cont'd)

| Compound No. | $R^1$ | $R^2$ | $^1$H-NMR (δ value)* |
|---|---|---|---|
| 104 | m-Cl | p-$CF_3$ | 9.5 (s, 1H), 8.7-8.55 (d, 2H), 7.85-7.2 (m, 6H), 2.8 (s, 3H) |
| 105 | m-Br | H | 9.25 (s, 1H), 8.55-7.3 (m, 9H), 3.15 (s, 3H) |
| 106 | m-Br | p-Cl | 9.45 (s, 1H), 8.55-7.5 (m, 8H), 3.2 (s, 3H) |
| 107 | m-Br | p-Br | 9.5 (s, 1H), 8.5-8.35 (d, 2H), 7.95-7.4 (m, 6H), 3.2 (s, 3H) |
| 108 | m-Br | p-$CF_3$ | 9.45 (s, 1H), 8.7-8.5 (d, 2H), 8.0-7.4 (m, 6H), 3.2 (s, 3H) |
| 109 | o-Br | p-$CF_3$ | 9.55 (s, 1H), 8.65-8.5 (d, 2H), 7.95-7.35 (m, 6H), 3.2 (s, 3H) |
| 110 | m-$CF_3$ | H | 9.5 (s, 1H), 8.65-7.4 (m, 9H), 2.8 (s, 3H) |
| 111 | m-$CF_3$ | p-$CH_3$ | 9.45 (s, 1H), 8.5-8.35 (d, 2H), 8.05-7.85 (m, 4H), 7.45-7.3 (d, 2H), 3.15 (s, 3H), 2.4 (s, 3H) |
| 112 | m-$CF_3$ | p-F | 9.45 (s, 1H), 8.7-7.0 (m, 8H), 3.0 (s, 3H) |
| 113 | m-$CF_3$ | p-Cl | 9.4 (s, 1H), 8.55-8.4 (d, 2H), 8.0-7.45 (m, 6H), 3.0 (s, 3H) |
| 114 | m-$CF_3$ | p-Br | 9.45 (s, 1H), 8.5-7.6 (m, 8H), 3.05 (s, 3H) |
| 115 | m-$CF_3$ | p-$CF_3$ | 9.5 (s, 1H), 8.75-7.6 (m, 8H), 2.8 (s, 3H) |

Table 3 (cont'd)

| Compound No. | $R^1$ | $R^2$ | $^1H$-NMR (δ value)* |
|---|---|---|---|
| 116 | m-$CF_3$ | p-$NO_2$ | 9.6 (s, 1H), 8.8-7.7 (m, 8H), 3.25 (s, 3H) |
| 117 | m-$CF_3$ | p-$CH_3O$ | 9.4 (s, 1H), 8.6-8.4 (d, 2H), 8.1-7.7 (m, 4H), 7.1-6.95 (d, 2H), 3.9 (s, 3H), 3.0 (s, 3H) |
| 118 | m-I | p-$CF_3$ | 9.5 (s, 1H), 8.75-8.6 (d, 2H), 8.15-7.2 (m, 6H), 3.22 (s, 1H) |
| 119 | m-F | H | 9.45 (s, 1H), 8.55-8.35 (m, 2H), 7.75-7.55 (m, 7H), 3.1 (s, 3H) |
| 120 | m-F | p-$CH_3$ | 9.35 (s, 1H), 8.4-8.25 (d, 2H), 7.6-7.3 (m, 6H), 3.2 (s, 3H), 2.45 (s, 3H) |
| 121 | m-F | p-F | 9.4 (s, 1H), 8.65-8.4 (m, 2H), 7.75-7.0 (m, 6H), 2.8 (s, 3H) |
| 122 | m-F | p-Cl | 9.35 (s, 1H), 8.5-8.35 (d, 2H), 7.7-7.5 (m, 6H), 3.2 (s, 3H) |
| 123 | m-F | p-Br | 9.35 (s, 1H), 8.4-8.25 (d, 2H), 7.75-7.4 (m, 6H), 3.15 (s, 3H) |
| 124 | m-I | p-F | 9.35 (s, 1H), 8.6-8.3 (m, 2H), 8.0-7.15 (m, 6H), 3.05 (s, 3H) |
| 125 | m-$CF_3O$ | p-$CF_3$ | 9.45 (s, 1H), 8.7-8.55 (d, 2H), 7.8-7.45 (m, 6H), 2.8 (s, 3H) |
| 126 | m-$CF_3O$ | p-F | 9.45 (s, 1H), 8.7-8.45 (m, 2H), 7.75-7.0 (m, 6H), 2.8 (s, 3H) |

Table 3 (cont'd)

| Compound No. | $R^1$ | $R^2$ | $^1H$-NMR (δ value)* |
|---|---|---|---|
| 127 | o-$CF_3$ | p-$CF_3$ | 9.45 (s, 1H), 8.7-8.55 (m, 2H), 7.8-7.65 (m, 6H), 2.8 (s, 3H) |
| 128 | m-$CH_3$ | p-F | 9.30 (s, 1H), 8.6-6.9 (m, 8H), 2.65 (s, 3H), 2.4 (s, 3H) |
| 129 | m-$C_2H_5O$ | p-F | 9.40 (s, 1H), 8.70-8.40 (m, 2H), 7.50-6.95 (m, 6H), 4.30-3.95 (q, 2H), 2.75 (s, 3H), 1.60-1.35 (t, 3H) |
| 130 | m-$CH_3OCH_2O$ | p-F | 9.35 (s, 1H), 8.60-8.35 (m, 2H), 7.45-6.95 (m, 6H), 5.20 (s, 2H), 3.45 (s, 3H), 2.75 (s, 3H) |
| 131 | m-$CF_3$ | o-F | 9.50 (s, 1H), 8.30-7.0 (m, 8H), 2.80 (s, 3H) |
| 132 | m-$C_6H_5O$ | p-F | 9.35 (s, 1H), 8.60-8.35 (m, 2H), 7.5-6.95 (m, 11H), 2.80 (s, 3H) |
| 133 | o-$CF_3$ | o-F | 9.5 (s, 1H), 8.20-7.0 (m, 8H), 2.80 (s, 3H) |
| 134 | m-Cl | o-F | 9.50 (s, 1H), 8.35-7.0 (m, 8H), 2.80 (s, 3H) |
| 135 | m-$CF_3$ | m-$CH_3O$ | 9.40 (s, 1H), 8.20-6.95 (m, 8H), 3.85 (s, 3H), 2.75 (s, 3H) |
| 136 | m-$CF_3$ | m-F | 9.45 (s, 1H), 8.40-7.05 (m, 8H), 2.80 (s, 3H) |
| 137 | m-$CF_3$ | m-$CF_3$ | 9.45 (s, 1H), 8.85-8.60 (m, 2H), 8.10-7.40 (m, 6H), 2.75 (s, 3H) |

Table 3 (cont'd)

| Compound No. | $R^1$ | $R^2$ | $^1$H-NMR (δ value)* |
| --- | --- | --- | --- |
| 138 | m-$CF_3$O | m-$CF_3$ | 9.30 (s, 1H), 8.70-8.50 (m, 2H), 7.80-7.20 (m, 6H), 2.90 (s, 3H) |
| 139 | m-$CH_3OCH_2$O | p-$CF_3$ | 9.50 (s, 1H), 8.80-8.50 (m, 2H), 7.85-7.20 (m, 6H), 5.25 (s, 2H), 3.50 (s, 3H), 2.80 (s, 3H) |
| 140 | m-$CF_3$O | m-Cl | 9.40 (s, 1H), 8.60-8.35 (m, 2H), 7.90-7.25 (m, 6H), 2.75 (s, 3H) |
| 141 | m-$CF_3$ | p-$SCH_3$ | 9.30 (s, 1H), 8.45-7.15 (m, 8H), 2.75 (s, 3H), 2.50 (s, 3H) |
| 142 | m-$CH_3CH_2$O | p-$CF_3$ | 9.35 (s, 1H), 8.60-8.45 (m, 2H), 7.70-6.95 (m, 6H), 4.25-3.90 (q, 2H), 2.75 (s, 3H), 1.55-1.30 (t, 3H) |
| 143 | m-CN | p-$CF_3$ | 9.40 (s, 1H), 8.75-7.6 (m, 8H), 3.0 (s, 3H) |

*Solvent

$CDCl_3$: Compounds 104, 110, 112, 121, 125, 126, 127, 129-141

$CDCl_3$+DMSO-$d_6$: Compounds 113, 117, 128, 142, 143

DMSO-$d_6$: other compounds

The compound represented by the formula (VIII) is prepared by reacting a compound of the formula:

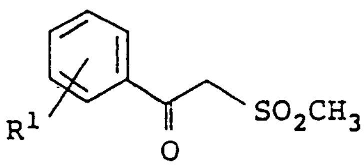

(X)

[wherein $R^1$ is as defined above] with N,N-dimethylformamide dimethylacetal. The reaction is usually carried out with or without a solvent at a temperature of 20 to 150 °C for a period of about 0.5 to 10 hours.

Normally, N,N-dimethylformamide dimethylacetal is used in an amount of 1.0 to 1.5 equivalents to one equivalent of the compound (X).

The solvent includes aliphatic hydrocarbons (e.g., hexane, heptane, ligroin, petroleum ether), aromatic hydrocarbons (e.g., benzene, toluene, xylene), halogenated hydrocarbons (e.g., chloroform, carbon tetrachloride, dichloroethane, chlorobenzene, dichlorobenzene), ethers (e.g., diethyl ether, diisopropyl ether, dioxane, tetrahydrofuran, diethylene glycol dimethyl ether), alcohols (e.g., methanol, ethanol, isopropanol, t-butanol, octanol, cyclohexanol, methylcellosolve, diethylene glycol, glycerin), acid amides (e.g., N,N-dimethylformamide), sulfur compounds (e.g., dimethyl sulfoxide, sulfolane), and mixtures thereof.

Production Example 7

2.5 g of m-trifluoromethyl-α-methanesulfonylacetophenone and 1.4 g of N,N-dimethylformamide dimethylacetal were dissolved in 40 ml of toluene, and the resulting mixture was heated under reflux for 2 hours. The solvent was distilled away under reduced pressure. The residue was treated with column chromatography on silica gel, thereby obtaining 3 g of 1-(3-trifluoromethylbenzoyl)-1-methanesulfonyl-2-(N,N-dimethylamino)ethene (Compound 205).

Table 4 illustrates examples of the compound (VIII) produced according to the above Production Example 7.

Table 4

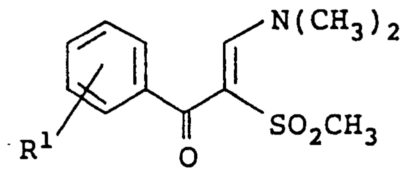

| Compound No. | $R^1$ | $^1H$-NMR (δ value, $CDCl_3$ solvent) |
|---|---|---|
| 201 | m-F | 7.8 (s, 1H), 7.7-7.2 (m, 4H), 3.2 (s, 3H), 2.8 (brs, 6H) |
| 202 | m-Cl | 7.7-7.1 (m, 5H), 3.05 (s, 3H), 2.7 (brs, 6H) |
| 203 | m-Br | 7.9-7.15 (m, 5H), 3.15 (s, 3H), 2.75 (brs, 6H) |
| 204 | m-I | 8.15-7.1 (m, 5H), 3.2 (s, 3H), 2.8 (brs, 6H) |
| 205 | m-$CF_3$ | 8.0-7.5 (m, 5H), 3.15 (s, 3H), 2.8 (brs, 6H) |
| 206 | o-Br | 7.85-7.25 (m, 5H), 3.0 (brs, 9H) |
| 207 | m-$CF_3O$ | 7.80-7.40 (m, 5H), 3.15 (s, 3H), 2.75 (brs, 6H) |
| 208 | m-$CH_3OCH_2O$ | 7.70 (s, 1H), 7.40-7.10 (m, 4H), 5.15 (s, 2H), 3.40 (s, 3H), 3.15 (s, 3H), 2.7 (brs, 6H) |

The compound represented by the formula (IV) is prepared by reacting a compound of the formula:

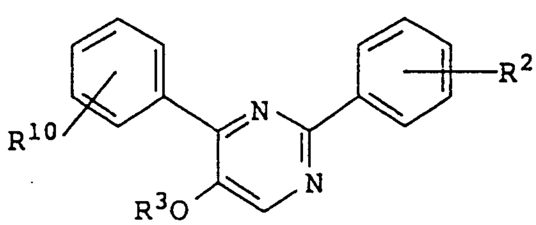

(I-5)

[wherein $R^{10}$ is a methoxymethoxy group at the ortho or meta position; and $R^2$ and $R^3$ are each as defined above] with an acid. The reaction is usually carried out with a solvent at a temperature of 20 to 100 °C for a period of 1 to 5 hours.

Normally, the acid is used in an amount of 1 to 10 equivalents to one equivalent of the compound (I-5).

The solvent includes aliphatic hydrocarbons (e.g., hexane, heptane, ligroin, petroleum ether), aromatic hydrocarbons (e.g., benzene, toluene, xylene), halogenated hydrocarbons (e.g., chlorobenzene, dichlorobenzene), ethers (e.g., diethyl ether, diisopropyl ether, dioxane, tetrahydrofuran, diethylene glycol dimethyl ether), ketones (e.g., acetone, methyl ethyl ketone, methyl isobutyl ketone, isophorone, cyclohexanone), alcohols (e.g., methanol, ethanol, isopropanol, t-butanol), water, and mixtures thereof.

The acid includes organic acids (e.g., acetic acid), inorganic acids (e.g., hydrochloric acid, sulfuric acid), and Lewis acids (e.g., fluoroborane ether complex).

After completion of the reaction, the reaction mixture is subjected to the usual after-treatment such as extraction with organic solvents, concentration, etc., and if necessary, purified by chromatography, recrystallization, etc. to obtain the desired compound.

Production Example 8

8 g of 2-(4-fluorophenyl)-4-(3-methoxymethoxyphenyl)-5-methoxypyrimidine was added to a mixture of 150 ml of tetrahydrofuran and 150 ml of isopropyl alcohol. After adding 10 ml of 12 N hydrochloric acid thereto, the mixture was stirred for 3 hours at 60 °C. The solvent was distilled away under reduced pressure. The residue was washed with water, and then dried to obtain 7 g of 2-(4-fluorophenyl)-4-(3-hydroxyphenyl)-5-methoxypyrimidine.

For the practical use of the compound (I), it is usually formulated with conventional solid or liquid carriers or diluents as well as surface active agents or auxiliary agents into conventional preparation forms such as emulsifiable concentrates, wettable powders, suspensions and granules. The content of the compound (I) as the active ingredient in such preparation forms is normally within a range of about 1 to 80% by weight, preferably of about 2 to 70% by weight. Examples of the solid carrier or diluent are fine powders of granules of kaolin clay, attapulgite clay, bentonite, terra alba, pyrophyllite, talc, diatomaceous earth, calcite, walnut shell powders, urea, ammonium sulfate and synthetic hydrous silicate, etc. As the liquid carrier or diluent, there may be exemplified aromatic hydrocarbons (e.g., xylene, methylnaphthalene), alcohols (e.g., isopropanol, ethylene glycol, cellosolve), ketones (e.g., acetone, cyclohexanone, isophorone), soybean oil, cotton seed oil, dimethyl sulfoxide, N,N-dimethylformamide, acetonitrile and water.

The surface active agent used for emulsification, dispersion or spreading may be of any type, for instance, either anionic or non-ionic. Examples of the surface active agent include alkylsulfates, alkylsulfonates, alkylarylsulfonates, dialkylsulfosuccinates, phosphates of polyoxyethylenealkylaryl ethers, polyoxyethylene alkyl ethers, polyoxyethylene alkylaryl ethers, polyoxyethylene-polyoxypropylene block copolymer, sorbitan fatty acid esters and polyoxyethylene sorbitan fatty acid esters. Examples of the auxiliary agents include ligninsulfonates, sodium alginate, polyvinyl alcohol, gum arabic , carboxymethyl cellulose and isopropyl acid phosphate.

Practical embodiments of the herbicidal composition according to the present invention are illustratively shown in the following Formulation Examples wherein parts are by weight. The compound number of the active ingredient corresponds to the one in Table 2.

Formulation Example 1

Seventy parts of Compound 8 or 17, 3 parts of calcium ligninsulfonate, 2 parts of sodium laurylsulfate and 25 parts of synthetic hydrous silicate are well mixed while being powdered to obtain a wettable powder.

Formulation Example 2

Ten parts of Compound 10, 18, or 20, 14 parts of polyoxyethylene styrylphenyl ether, 6 parts of calcium dodecylbenzenesulfonate, 70 parts of xylene are well mixed to obtain an emulsifiable concentrate.

Formulation Example 3

Two parts of Compound 12 or 23, 1 part of synthetic hydrous silicate, 2 parts of calcium ligninsulfonate, 30 parts of bentonite and 65 parts of kaolin clay are well mixed while being powdered. The mixture is then kneaded with water, granulated and dried to obtain granules.

Formulation Example 4

Twenty-five parts of Compound 20 is mixed with 3 parts of polyoxyethylene sorbitan monooleate, 3 parts of carboxymethyl cellulose and 69 parts of water and pulverized until the particle size of the mixture becomes less than 5 microns to obtain a suspension.

The compound (I) thus formulated in any suitable preparation form is useful for pre-emergence or post-emergence control of undesired weeds by soil or foliar treatment as well as flood following treatment. These treatments include application to the soil surface prior to or after transplanting, incorporation into the soil, etc. The foliar treatment may be effected by spraying the herbicidal composition containing the compound (I) over the top of plants. It may also be applied directly to the weeds if care is taken to keep the chemical off the crop foliage.

The compound (I) may be used together with any other herbicide to improve its activity as a herbicide, and in some cases, a synergistic effect can be expected. Further, it may be applied in combination with an insecticide, an acaricide, a nematocide, a fungicide, a plant growth regulator, a fertilizer, a soil improver, etc. Furthermore, it may be used as a herbicide applicable to agricultural plowed fields as well as paddy fields. It is also useful as a herbicide to be employed for orchards, pasture lands, lawns, forests, non-agricultural fields, etc.

The dosage of the compound (I) may vary depending on the prevailing weather conditions, the formulation used, the prevailing season, the mode of application, the soil involved, the crop and weed specides, etc. Generally, however, the dosage of the active ingredient is from about 1 to 8000 grams, preferably from about 5 to 2000 grams per hectare.

The herbicidal composition of the invention formulated in the form of an emulsifiable concentrate, a wettable powder or a suspension may ordinarily be employed by diluting it with water at a volume of about 100 to 1000 liters per hectare, if necessary, with addition of an auxiliary agent such as a spreading agent. Examples of the spreading agent include, in addition to the surface active agents as noted above, polyoxyethylene resin acid (ester), ligninsulfonate, abietylenic acid salt, dinaphthylmethanedisulfonate, paraffin, etc. The composition formulated in the form of granules may be normally applied as such without dilution.

The biological data of the compound (I) as herbicides will be illustratively shown in the following Test Examples wherein the phytotoxicity to crop plants and the herbicidal activity on weeds were observed visually as to the degree of germination as well as the growth inhibition and rated with an index 0, 1, 2, 3, 4, 5, 6, 7, 8, 9, or 10, the numeral "0" indicating no material difference as seen in comparison with the untreated plants and the numeral "10" indicating the complete inhibition or death of the test plants.

The compounds as shown in Table 5 were used for comparison.

Table 5

| Compound Code | Structural Formula | Remarks |
|---|---|---|
| A | 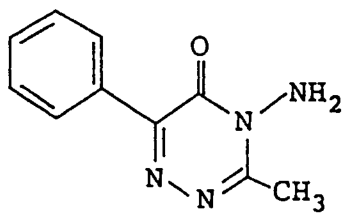 | Metamitron (Commercial herbicide) |
| B | 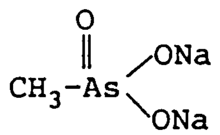 | DSMA (Commercial herbicide) |
| C | 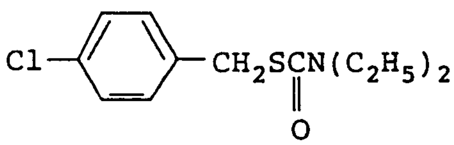 | Benthiocarb (Commercial herbicide) |

Table 5 (cont'd)

| Compound Code | Structural Formula | Remarks |
|---|---|---|
| D | 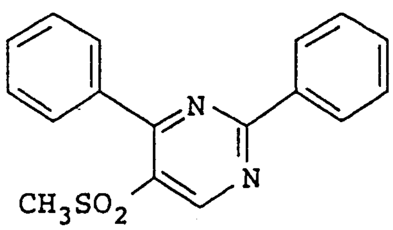 | Described in J. Heterocyclic Chem., 23, 77 (1986) |

Test Example 1

Cylindrical plastic pots (diameter, 10 cm; height, 10 cm) were filled with upland field soil, and the seeds of tall morningglory and velvetleaf were sowed therein and covered with soil. A designated amount of the test compound formulated in a wettable powder as in Formulation Example 1 was diluted with water, and the dilution was sprayed onto the soil surface by means of a small hand sprayer at a spray volume of 1000 liters per hectare. The test plants were grown in a greenhouse for 20 days, and the herbicidal activity was examined. The results are shown in Table 6.

Table 6

| Compound No. | Dosage (g/ha) | Herbicidal Activity | |
|---|---|---|---|
| | | Tall Morningglory | Velvetleaf |
| 8 | 2000 | 10 | 10 |
| 12 | 2000 | 10 | - |
| 16 | 2000 | 10 | 10 |
| 17 | 2000 | 10 | 9 |
| | 500 | 10 | - |
| 18 | 2000 | 9 | 10 |
| | 500 | - | 10 |
| 19 | 8000 | 10 | 10 |
| 21 | 2000 | 10 | 10 |
| 31 | 2000 | 10 | 10 |
| | 500 | 10 | - |
| 32 | 2000 | 10 | 10 |
| 34 | 2000 | 10 | 9 |
| | 500 | 10 | - |
| 58 | 2000 | 10 | - |
| 61 | 2000 | 9 | - |
| 65 | 2000 | 10 | - |
| A | 8000 | 6 | 6 |
| | 2000 | 2 | 2 |
| D | 2000 | 0 | 0 |

Test Example 2

Cylindrical plastic pots (diameter, 10 cm; height, 10 cm) were filled with upland field soil, and the seeds of tall morningglory, radish and velvetleaf were sowed therein and cultivated in a greenhouse for 10 days. A designated amount of the test compound formulated in an emulsifiable concentrate as in Formulation Example 2 was diluted with water containing a spreading agent, and the dilution was sprayed over the foliage of the test plant by means of a small hand sprayer at a spray volume of 1000 liters per hectare. The test plants were further grown in a greenhouse for 20 days, and the herbicidal activity was examined. The results are shown in Table 7.

Table 7

| Compound No. | Dosage (g/ha) | Herbicidal Activity Radish | Velvet-leaf | Tall Morningglory |
|---|---|---|---|---|
| 2 | 500 | 8 | 8 | 9 |
| 6 | 2000 | 10 | 10 | 10 |
| | 500 | 9 | - | - |
| 7 | 2000 | 10 | 10 | 10 |
| | 500 | 9 | 9 | - |
| 8 | 2000 | 10 | 10 | 10 |
| | 500 | 10 | 9 | 10 |
| 9 | 500 | 10 | 10 | 10 |
| | 125 | 8 | - | 10 |
| 10 | 500 | 9 | 9 | 10 |
| | 125 | 9 | 9 | 9 |
| | 32 | 9 | - | 9 |

Table 7 (cont'd)

| Compound No. | Dosage (g/ha) | Herbicidal Activity Radish | Velvet-leaf | Tall Morningglory |
|---|---|---|---|---|
| 11 | 500 | - | 9 | 9 |
| 12 | 500 | 10 | 10 | 10 |
| | 125 | 9 | 9 | 10 |
| | 32 | - | 8 | 10 |
| 14 | 2000 | 9 | 10 | 9 |
| 16 | 500 | 10 | 9 | 10 |
| | 125 | 8 | 8 | 10 |
| 17 | 500 | 8 | 9 | 10 |
| | 125 | - | 9 | 10 |
| 18 | 500 | 10 | 10 | 10 |
| | 125 | 10 | 10 | 10 |
| | 32 | 10 | 9 | 9 |
| 19 | 2000 | 9 | 9 | 10 |
| | 500 | 9 | 9 | 10 |
| 20 | 500 | 10 | 9 | 10 |
| | 125 | 9 | 9 | 9 |
| | 32 | 9 | 8 | 9 |
| 21 | 500 | 10 | 9 | 9 |
| | 125 | 10 | 9 | 9 |
| | 32 | 10 | 9 | 9 |
| 23 | 500 | 10 | 8 | 10 |
| | 125 | 9 | 8 | 9 |
| 31 | 500 | 10 | 10 | 9 |
| | 125 | 10 | 9 | 9 |
| | 32 | 10 | 9 | 9 |
| 32 | 500 | 10 | 9 | 10 |
| | 125 | 10 | 9 | 10 |
| | 32 | 10 | 9 | 9 |

Table 7 (cont'd)

| Compound No. | Dosage (g/ha) | Herbicidal Activity Radish | Velvet-leaf | Tall Morningglory |
|---|---|---|---|---|
| 34 | 500 | 10 | 9 | 10 |
| | 125 | 9 | 9 | 10 |
| 47 | 2000 | 10 | 10 | 10 |
| | 500 | 9 | 10 | 10 |
| | 125 | 9 | 10 | 9 |
| 48 | 2000 | 10 | 9 | 10 |
| | 500 | 10 | 9 | 10 |
| | 125 | 10 | 9 | 10 |
| 49 | 2000 | 10 | 10 | 10 |
| | 500 | 10 | 9 | 10 |
| | 125 | 10 | 9 | 10 |
| 52 | 2000 | 9 | – | 9 |
| 58 | 2000 | 10 | 9 | 10 |
| | 500 | 9 | 9 | 10 |
| 60 | 2000 | 9 | 9 | – |
| | 500 | 9 | 9 | – |
| 61 | 500 | 10 | 9 | 10 |
| | 125 | 9 | 9 | 10 |
| | 32 | 9 | 8 | 10 |
| 62 | 500 | 9 | 9 | 10 |
| | 125 | 9 | 9 | 9 |
| 63 | 500 | 9 | 8 | 10 |
| | 125 | 9 | 8 | 9 |
| | 32 | 9 | 8 | 9 |
| 64 | 2000 | 9 | 9 | 9 |
| | 500 | 9 | 9 | 9 |
| 65 | 500 | 10 | 9 | 10 |
| | 125 | 10 | 9 | 10 |
| | 32 | 10 | 9 | 10 |

Table 7 (cont'd)

| Compound No. | Dosage (g/ha) | Herbicidal Activity Radish | Velvet-leaf | Tall Morningglory |
|---|---|---|---|---|
| 66 | 500 | 9 | 9 | 10 |
| | 125 | 9 | 9 | 10 |
| | 32 | 9 | 9 | 9 |
| B | 8000 | 9 | 0 | 0 |
| | 2000 | 8 | 0 | 0 |
| D | 2000 | 3 | 2 | - |

Test Example 3

Cylindrical plastic pots (diameter, 8 cm; height, 12 cm) were filled with paddy field soil, and the seeds of barnyardgrass (Echinochloa oryzicola) were sowed in 1 to 2 cm depth, and water was poured therein to make a flooded condition.

Five days (at that time weeds began to germinate) thereafter, a designated amount of the test compound formulated in an emulsifiable concentrate as in Formulation Example 2 and diluted with water (5 ml) was applied to the pots by perfusion. The test plants were grown for an additional 20 days in a greenhouse, and the herbicidal activity was examined. The results are shown in Table 8.

Table 8

| Compound No. | Dosage (g/ha) | Herbicidal Activity Banyardgrass |
|---|---|---|
| 2 | 4000 | 10 |
| 4 | 4000 | 10 |
| 6 | 4000 | 10 |
| 7 | 4000 | 10 |
| 8 | 4000 | 10 |
| 9 | 4000 | 10 |
| 10 | 4000 | 10 |
| 11 | 4000 | 10 |
| 12 | 4000 | 10 |
| 13 | 4000 | 10 |
| 14 | 4000 | 10 |
| 15 | 4000 | 10 |
| 16 | 4000 | 10 |
| 17 | 4000 | 10 |
| 18 | 4000 | 10 |
| 19 | 4000 | 10 |
| 20 | 4000 | 10 |
| 21 | 4000 | 10 |
| 23 | 4000 | 10 |
| 31 | 4000 | 10 |
| 32 | 4000 | 10 |
| 34 | 4000 | 10 |

Table 8 (cont'd)

| Compound No. | Dosage (g/ha) | Herbicidal Activity Banyardgrass |
|---|---|---|
| 47 | 4000 | 10 |
| 48 | 4000 | 10 |
| 49 | 4000 | 10 |
| 52 | 4000 | 10 |
| 58 | 4000 | 10 |
| 61 | 4000 | 10 |
| 62 | 4000 | 10 |
| 64 | 4000 | 10 |
| 65 | 4000 | 10 |
| 66 | 4000 | 10 |
| D | 4000 | 0 |

Test Example 4

Wagner's pots (1/5000 are) were filled with paddy field soil, and the seeds of barnyardgrass (Echinochloa oryzicola), broad-leaved weeds (i.e., common falsepimpernel, indian toothcup, waterwort), and hardstem bulrush were sowed in 1 to 2 cm depth. Water was poured therein to make a flooded condition, and rice seedlings of 3-leaf stage and tubers of water nutgrass were transplanted therein, and the test plants were grown in a greenhouse. Five days (at that time barnyardgrass began to germinate) thereafter, a designated amount of the test compound formulated in an emulsifiable concentrate as in Formulation Example 2 and diluted with water (10 ml) was applied to the pots by perfusion. The test plants were grown for an additional 20 days in the greenhouse, and the herbicidal activity was examined. The results are shown in Table 9. At the time of the treatment, the depth of water in the pots was kept at 4 cm and following two days, water was let to leak in a volume corresponding to a 3 cm depth per day.

Table 9

| Compound No. | Dosage (g/ha) | Phytotoxicity Rice Plant | Herbicidal Activity Barnyard-grass | Herbicidal Activity Broad-leaved Weeds | Herbicidal Activity Hardstem Bulrush | Herbicidal Activity Water Nutgrass |
|---|---|---|---|---|---|---|
| 18 | 250 | 3 | 10 | 10 | 10 | 10 |
| | 63 | 0 | 10 | 10 | 10 | 10 |
| 19 | 250 | 1 | 10 | 10 | 9 | 10 |
| | 63 | 0 | 10 | 10 | 8 | 9 |
| 21 | 250 | 2 | 10 | 10 | 10 | 10 |
| | 63 | 0 | 10 | 10 | 10 | 10 |
| C | 250 | 0 | 4 | 0 | 0 | 0 |
| | 63 | 0 | 0 | 0 | 0 | 0 |

## Claims

1. A compound of the formula:

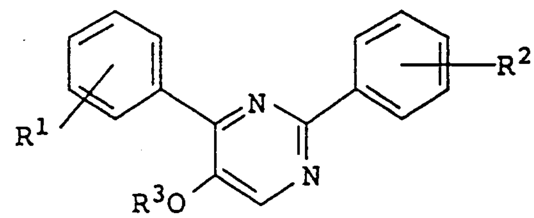

wherein $R^1$ represents hydrogen atom, or a halogen atom, a ($C_1$ to $C_3$)-alkyl group, a halo-($C_1$ to $C_3$)-alkyl group, a ($C_1$ to $C_6$)-alkoxy group which may contain an unsaturated bond, a ($C_1$ to $C_2$)-alkylthio group, a halo-($C_1$ to $C_6$)-alkoxy group which may contain an unsaturated bond, a halo-($C_1$ to $C_2$)-alkylthio group, a phenoxy group, a ($C_1$ to $C_3$)-alkylcarboxy group, a halo-($C_1$ to $C_3$)-alkylcarboxy group, a ($C_1$ to $C_2$)-alkoxy-($C_1$ to $C_2$)-alkoxy group, a ($C_1$ to $C_3$)-alkylsulfonyloxy group, a halo-($C_1$ to $C_3$)-alkylsulfonyloxy group, a cyano group, a ($C_1$ to $C_3$)-alkoxycarbonyl group, a hydroxycarbonyl group, an aminomethyl group or a hydroxymethyl group at the ortho or meta position; $R^2$ represents a hydrogen atom, a halogen atom, a ($C_1$ to $C_2$)-alkyl group, a halo-($C_1$ to $C_2$)-alkyl group, a ($C_1$ to $C_2$)-alkoxy group, a nitro group, a ($C_1$ to $C_2$)-alkylthio group, a halo-($C_1$ to $C_2$)-alkylthio group or a halo-($C_1$ to $C_2$)-alkoxy group; and $R^3$ represents a ($C_1$ to $C_2$)-alkyl group, provided that both $R^1$ and $R^2$ are not a hydrogen atom at the same time.

2. A compound according to claim 1, wherein $R^1$ represents a hydrogen atom, or a halogen atom, a ($C_1$ to $C_3$)-alkyl group, a halo-($C_1$ to $C_3$)-alkyl group, a ($C_1$ to $C_6$)-alkoxy group which may contain an unsaturated bond, a ($C_1$ to $C_2$)-alkylthio group, a halo-($C_1$ to $C_6$)-alkoxy group which may contain an unsaturated bond, a halo-($C_1$ to $C_2$)-alkylthio group, a phenoxy group, a ($C_1$ to $C_3$)-alkylcarboxy group, a halo-($C_1$ to $C_3$)-alkylcarboxy group, a ($C_1$ to $C_2$)-alkoxy-($C_1$ to $C_2$)-alkoxy group, a ($C_1$ to $C_3$)-alkylsulfonyloxy group, a halo-($C_1$ to $C_3$)-alkylsulfonyloxy group or a cyano group at the meta position; and $R^2$ represents a hydrogen atom, or a halogen atom, a ($C_1$ to $C_2$)-alkyl group, a halo-($C_1$ to $C_2$)-alkyl group or a ($C_1$ to $C_2$)-alkoxy group at the meta or para position.

3. A compound according to claim 1, wherein $R^1$ represents a hydrogen atom, or a halogen atom, a trifluoromethyl group or a trifluoromethoxy group at the meta position; $R^2$ represents a hydrogen atom, or a halogen atom or a trifluoromethyl group at the para position; and $R^3$ represents a methyl group.

4. A compound according to claim 3, wherein $R^1$ represents a trifluoromethyl group or a trifluoromethoxy group at the meta position; $R^2$ represents a fluorine atom, a chlorine atom or a trifluoromethyl group at the para position; and $R^3$ represents a methyl group.

5. 2-(4-Fluorophenyl)-4-(3-trifluoromethylphenyl)-5-methoxypyrimidine.

6. 2-(4-Trifluoromethylphenyl)-4-(3-trifluoromethoxyphenyl)-5-methoxypyrimidine.

7. 2-(4-Fluorophenyl-4-(3-trifluoromethoxyphenyl)-5-methoxypyrimidine.

8. A process for preparing a compound of the formula:

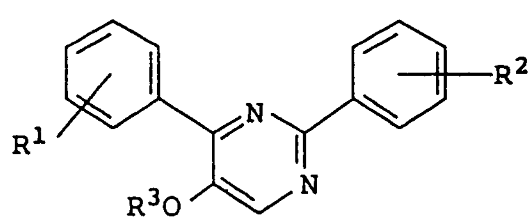

wherein $R^1$, $R^2$ and $R^3$ are each as defined in Claim 1,2,3 or 4 which comprises reacting a compound of the formula:

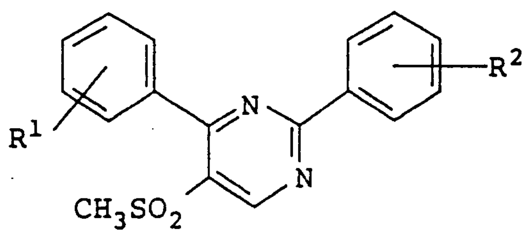

wherein $R^1$ and $R^2$ are each as defined in claim 1, 2, 3 or 4 with a compound of the formula:

$R^3OM$

wherein M is a metal atom, and $R^3$ is as defined in claim 1, 2, 3 or 4.

**9.** A compound of the formula:

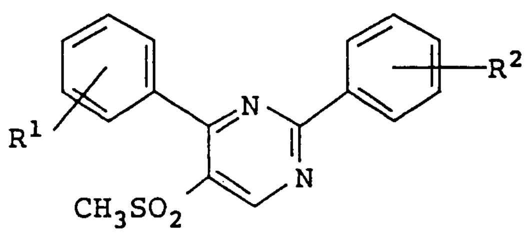

wherein $R^1$ and $R^2$ are each as defined in claim 1, 2, 3 or 4.

**10.** A herbicidal composition which comprises as an active ingredient a herbicidally effective amount of the compound according to any of claims 1 to 7, and an inert carrier or a diluent.

**11.** A method for controlling undesired weeds, which comprises applying a herbicidally effective amount of the compound according to any of claims 1 to 7 and an inert carrier or a diluent to the area where undesired weeds grow or will grow.

**12.** Use of a compound according to any of claims 1 to 7 as a herbicide.

**Patentansprüche**

**1.** Verbindung der Formel:

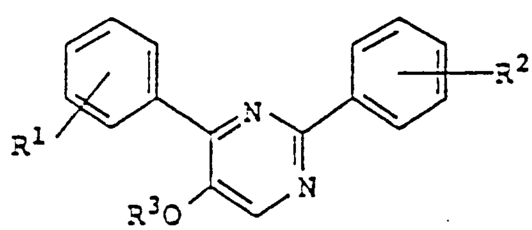

worin $R^1$ ein Wasserstoffatom oder ein Halogenatom, eine $(C_1-C_3)$-Alkylgruppe, eine Halogen-$(C_1-C_3)$-alkylgruppe, eine $(C_1-C_6)$-Alkoxygruppe, die eine Mehrfachbindung aufweisen kann, eine $(C_1-C_2)$-Alkylthiogruppe, eine Halogen-$(C_1-C_6)$-alkoxygruppe, die eine Mehrfachbindung enthalten kann, eine

Halogen-($C_1$-$C_2$)-alkylthiogruppe, eine Phenoxygruppe, eine ($C_1$-$C_3$)-Alkylcarboxygruppe, eine Halogen-($C_1$-$C_3$)-alkylcarboxygruppe, eine ($C_1$-$C_2$)-Alkoxy-($C_1$-$C_2$)-alkoxygruppe, eine ($C_1$-$C_3$)-Alkylsulfonyloxygruppe, eine Halogen-($C_1$-$C_3$)-alkylsulfonyloxygruppe, eine Cyanogruppe, eine ($C_1$-$C_3$)-Alkoxycarbonylgruppe, eine Hydroxycarbonylgruppe, eine Aminomethylgruppe oder eine Hydroxymethylgruppe in der Ortho- oder Meta-Stellung; $R^2$ ein Wasserstoffatom, ein Halogenatom, eine ($C_1$-$C_2$)-Alkylgruppe, eine Halogen-($C_1$-$C_2$)-alkylgruppe, eine ($C_1$-$C_2$)-Alkoxygruppe, eine Nitrogruppe, eine ($C_1$-$C_2$)-Alkylthiogruppe, eine Halogen-($C_1$-$C_2$)-alkylthiogruppe oder eine Halogen-($C_1$-$C_2$)-alkoxygruppe und $R^3$ eine ($C_1$-$C_2$)-Alkylgruppe bedeutet, mit der Massgabe, dass $R^1$ und $R^2$ nicht gleichzeitig ein Wasserstoffatom bedeuten.

2. Verbindung nach Anspruch 1, worin $R^1$ ein Wasserstoffatom oder ein Halogenatom, eine ($C_1$-$C_3$)-Alkylgruppe, eine Halogen-($C_1$-$C_3$)-alkylgruppe, eine ($C_1$-$C_6$)-Alkoxygruppe, die eine Mehrfachbindung aufweisen kann, eine ($C_1$-$C_2$)-Alkylthiogruppe, eine Halogen-($C_1$-$C_6$)-alkoxygruppe, die eine Mehrfachbindung enthalten kann, eine Halogen-($C_1$-$C_2$)-alkylthiogruppe, eine Phenoxygruppe, eine ($C_1$-$C_3$)-Alkylcarboxygruppe, eine Halogen-($C_1$-$C_3$)-alkylcarboxygruppe, eine ($C_1$-$C_2$)-Alkoxy-($C_1$-$C_2$)-alkoxygruppe, eine ($C_1$-$C_3$)-Alkylsulfonyloxygruppe, eine Halogen-($C_1$-$C_3$)-alkylsulfonyloxygruppe oder eine Cyanogruppe in Meta-Stellung; und $R^2$ ein Wasserstoffatom, oder ein Halogenatom, eine ($C_1$-$C_2$)-Alkylgruppe, eine Halogen-($C_1$-$C_2$)-alkylgruppe oder eine ($C_1$-$C_2$)-Alkoxygruppe in der Meta- oder Para-Stellung bedeutet.

3. Verbindung nach Anspruch 1, worin $R^1$ ein Wasserstoffatom oder ein Halogenatom, eine Trifluormethylgruppe oder eine Trifluormethoxygruppe in der Meta-Stellung; $R^2$ ein Wasserstoffatom oder ein Halogenatom oder eine Trifluormethylgruppe in der Para-Stellung und $R^3$ eine Methylgruppe bedeutet.

4. Verbindung nach Anspruch 3, worin $R^1$ eine Trifluormethylgruppe oder eine Trifluormethoxygruppe in der Meta-Stellung; $R^2$ ein Fluoratom, ein Chloratom oder eine Trifluormethylgruppe in der Para-Stellung und $R^3$ eine Methylgruppe bedeutet.

5. 2-(4-Fluorphenyl)-4-(3-trifluormethylphenyl)-5-methoxypyrimidin.

6. 2-(4-Trifluormethylphenyl)-4-(3-trifluormethoxyphenyl)-5-methoxypyrimidin.

7. 2-(4-Fluorphenyl)-4-(3-trifluormethoxyphenyl)-5-methoxypyrimidin.

8. Verfahren zur Herstellung einer Verbindung der Formel:

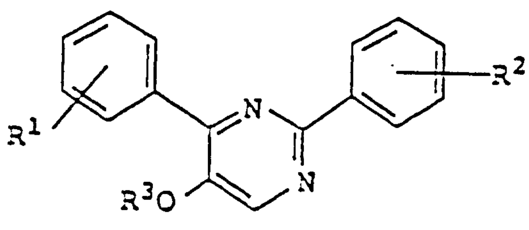

worin $R^1$, $R^2$ und $R^3$ die in Anspruch 1, 2, 3 oder 4 angegebene Bedeutung haben, durch Umsetzung einer Verbindung der Formel:

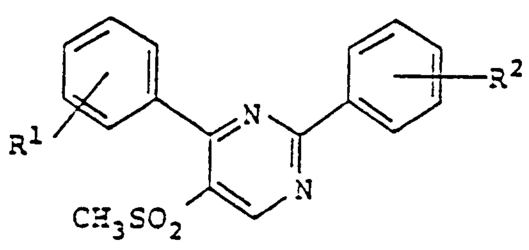

worin $R^1$ und $R^2$ die in Anspruch 1, 2, 3 oder 4 angegebene Bedeutung haben mit einer Verbindung der Formel:

$R^3OM$

worin M ein Metallatom und $R^3$ die in Anspruch 1, 2, 3 oder 4 angegebene Bedeutung hat.

**9.** Verbindung der Formel:

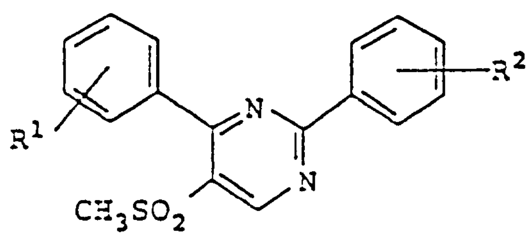

worin $R^1$ und $R^2$ die in Anspruch 1, 2, 3 oder 4 angegebene Bedeutung haben.

**10.** Herbizide Zusammensetzung enthaltend als aktive Komponente eine herbizid wirksame Menge eine Verbindung nach einem der Ansprüche 1 bis 7 und einen innerten Träger oder Verdünner.

**11.** Verfahren zur Bekämpfung von unerwünschten Pflanzen, durch Aufbringen einer herbizid wirksamen Menge einer Verbindung nach einem der Ansprüche 1 bis 7 und eines innerten Trägers oder Verdünners auf das Gebiet, wo die unerwünschten Pflanzen wachsen oder wachsen werden.

**12.** Verwendung einer Verbindung nach einem der Ansprüche 1 bis 7 als Herbizid.

## Revendications

**1.** Composé de formule:

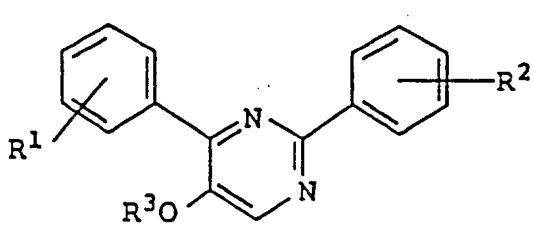

dans laquelle $R^1$ représente un atome d'hydrogène ou une atome d'halogène, un groupe alkyle ($C_1$ à $C_3$), un groupe alkyle-halo-($C_1$ à $C_3$), un groupe alkoxy-($C_1$ à $C_6$), qui peut contenir une liaison insaturée, un groupe alkylthio($C_1$ à $C_2$), un groupe alkoxy-halo-($C_1$ à $C_6$) qui peut contenir une liaison insaturée, un groupe alkylthio-halo-($C_1$ à $C_2$), un groupe phénoxy, un groupe alkylcarboxy- ($C_1$ à $C_3$), un groupe alkylcarboxy-halo-($C_1$ à $C_3$), une groupe alkoxy - ($C_1$ à $C_2$) - alkoxy - ($C_1$ à $C_2$), un groupe alkylsulfonyloxy- ($C_1$ à $C_3$), un groupe alkylsulfonyloxy-halo-($C_1$ à $C_3$), un groupe cyano, un groupe alkoxycarbonyle -($C_1$ à $C_3$), un groupe hydroxycarbonyle, un groupe aminométhyle ou un groupe hydroxyméthyle à la position ortho ou méta. $R^2$ représente un atome d'hydrogène, un atome d'halogène, un groupe alkyle - ($C_1$ à $C_2$), un groupe alkyle-halo-($C_1$ à $C_2$), un groupe alkoxy- ($C_1$ à $C_2$), un groupe nitro, un groupe alkylthio - ($C_1$ à $C_2$), un groupe alkylthio-halo-($C_1$ à $C_2$) ou un groupe alkoxy-halo-($C_1$ à $C_2$); et $R^3$ représente un groupe alkyle ($C_1$ à $C_2$), à condition que $R^1$ et $R^2$ ne soient pas simultanément un atome d'hydrogène.

**2.** Composé selon la revendication 7, dans lequel $R^1$ représente un atome d'hydrogène, ou un atome d'halogène, un groupe alkyle - ($C_1$ à $C_3$), un groupe alkyl-halo-($C_1$ à $C_3$), un groupe alkoxy-($C_1$ à $C_6$) qui peut contenir une liaison insaturée, un groupe alkylthio - ($C_1$ à $C_2$), un groupe alkoxy-halo-($C_1$ à $C_6$)

qui peut contenir une liaison insaturée, un groupe alkylthio-halo-($C_1$ à $C_2$), un groupe phénoxy, un groupe alkylcarboxy-($C_1$ à $C_3$), un groupe alkylcarboxy-halo-($C_1$ à $C_3$), un groupe alkoxy-($C_1$ à $C_2$)-alkoxy-($C_1$ à $C_2$), un groupe alkylsulfonyloxy-($C_1$ à $C_3$), un groupe alkylsulfonyloxy-halo-($C_1$ à $C_3$) ou un groupe cyano à la position méta; et $R^2$ représente un atome d'hydrogène, ou un atome d'halogène, une groupe alkyle ($C_1$ à $C_2$), un groupe alkyle-halo -($C_1$ à $C_2$) ou un groupe alkoxy-($C_1$ à $C_2$) à la position méta ou para.

**3.** Composé selon la revendication 1, dans lequel $R^1$ représente un atome d'hydrogène, ou un atome d'halogène, un groupe trifluorméthyle ou un groupe trifluorométhoxy à la position méta; $R^2$ représente un atome d'hydrogène, ou un atome d'halogène ou un groupe trifluorométhyle à la position para; et $R^3$ représente un groupe méthyle.

**4.** Composé selon la revendication 3, dans lequel $R^1$ représente un groupe trifluorométhyle ou un groupe trifluorométhoxy à la position méta; $R^2$ représente un atome de fluor, un atome de chlor ou un groupe trifluorométhyle à la position para; et $R^3$ représente un groupe méthyle.

**5.** 2-(4-Fluorophényle)-4-(3-trifluorométhyle-phényle)-5-méthoxypyrimidine.

**6.** 2-(4-Trifluorométhylphényle)-4-(3-trifluorométhoxyphényle)-5-méthoxypyrimidine.

**7.** 2-(4-Fluorophényle)-4-(3-trifluorométhoxyphényle)-5-méthoxypyrimidine.

**8.** Procédé pour la préparation d'un composé de formule:

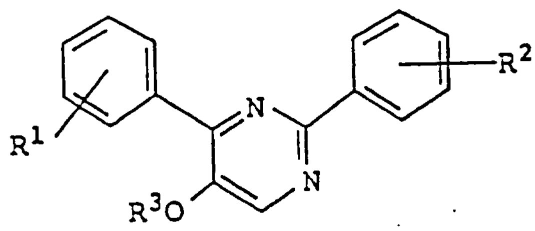

dans lequel $R^1$, $R^2$ et $R^3$ sont chacuns tels que définis dans les revendications 1,2,3 ou 4 qui comprend la reaction d'un composé de formule:

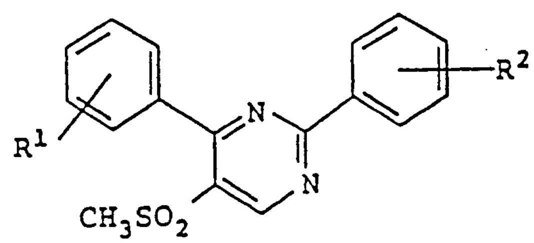

dans lequel $R^1$ et $R^2$ sont chacuns tels que définis dans les revendications 1, 2, 3 ou 4 avec un composé de formule:

$R^3OM$

dans lequel M est un atome métallique, et $R^3$ est tel que défini dans les revendications 1,2,3 ou 4.

**9.** Composé de formule:

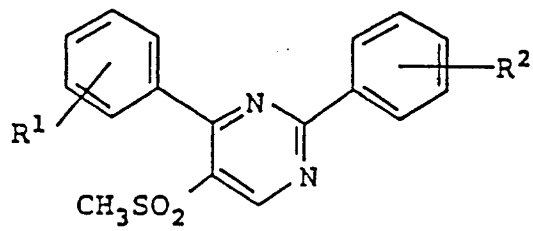

dans lequel $R^1$ et $R^2$ sont chacuns tels que définis dans les revendications 1,2,3 et 4.

**10.** Une composition herbicide qui comprend en tant qu'ingrédient actif une quantité efficace au point de vue herbicide du composé selon l'une des revendications 1 à 7 et un véhicule inerte ou un diluant.

**11.** Procédé pour combattre les mauvais herbes, qui comprend l'application d'une quantité efficace au point de vue herbicide du composé selon l'une des revendications 1 à 7 et un véhicule inert ou un diluant à un terrain où des mauvaises herbes poussent ou pousseront.

**12.** Utilisation d'un composé selon l'une des revendications 1 à 7 en tant qu'herbicide.